# EUROPEAN PATENT APPLICATION

(11) **EP 3 308 786 A1**
(43) Date of publication of application: **18.04.2018**
(21) Application number: 16193748.7
(22) Date of filing: 13.10.2016
(51) Int. Cl.: A61K 31/4439, A61K 31/496, A61K 31/51, A61P 31/12

(54) **FGFR REGULATON FOR THE TREATMENT OF VIRAL INFECTIONS**

(71) Applicant: ETH Zurich, 8092 Zurich (CH)
(72) Inventor: WERNER, Sabine, 8044 Zürich (CH); MEYER, Michael, 8032 Zürich (CH); MADDALUNO, Luigi, 8037 Zürich (CH)

(57) **Abstract**

The present invention is directed to a compound for inhibiting (i) FGF-R kinase activity or (ii) a component of the FGFR kinase signaling pathway for use in the therapeutic or prophylactic treatment of viral infections as well as corresponding pharmaceutical compositions for use in the same treatment and related methods of treatment.

SEQUENCE LISTING

<110>  ETH Zurich

<120>  FGFR REGULATON FOR THE TREATMENT OF VIRAL INFECTIONS

<130>  2016 056 01

<160>  4

<170>  PatentIn version 3.5

<210>  1
<211>  21
<212>  DNA
<213>  Artificial Sequence

<220>
<223>  primer for amplification of the genomic DNA for beta-actin

<400>  1
tactcctgct tgctgatcca c                                                21


<210>  2
<211>  20
<212>  DNA
<213>  Artificial Sequence

<220>
<223>  primer for amplification of the genomic DNA for beta-actin

<400>  2
tgtgtgggga gctgtcacat                                                  20


<210>  3
<211>  20
<212>  DNA
<213>  Artificial Sequence

<220>
<223>  primer for amplification of the genomic DNA for viral
       glycoprotein B

<400>  3
cgcatcaaga ccacctcctc                                                  20


<210>  4
<211>  15
<212>  DNA
<213>  Artificial Sequence

<220>
<223>  primer for amplification of the genomic DNA for viral
       glycoprotein B

<400>  4
gctcgcacca cgcga                                                       15

## Description

The present invention is directed to a compound for inhibiting (i) FGFR kinase activity or (ii) a component of the FGFR kinase signaling pathway for use in the therapeutic or prophylactic treatment of viral infections as well as corresponding pharmaceutical compositions for use in the same treatment and related methods of treatment.

### Background

Viral infections are widespread and a major health risk for humans and animals worldwide because they are generally difficult to treat. In most cases, the treatment of viral infections consists of reducing the symptoms by antipyretic and analgesic drugs, by avoiding exposure and reducing contamination, e.g. by disinfection. Also, there are vaccines for some viruses, which, of course, are only effective if administered prior to an infection.

For a limited number of viruses, virostatic medicaments are employed, which inhibit viral development. These virostatic agents, for example, interfere with the viral life cycle before cell entry, during viral synthesis or assembly or during the release phase from the host cell. A general draw-back of current virostatic medicaments is their virus-specific nature, their susceptibility to viral variation and/or their toxicity.

For example, the very common Herpes simplex virus (HSV) 1 affects the skin and the genital tract. Current treatment options involve the inhibition of the thymidine kinase of HSV by the administration of nucleoside analogs or the administration of Helicase-primase inhibitors, which are associated with certain toxicity, major side effects and mutagenic potential. Also, the virostatic treatment of HSV is presently limited to the systemic administration of the medicaments and there are no efficient topical or local treatment options.

Van et al. (Van ND et al., Gut 2016, 65, 1015-1023) investigated the modulation of hepatitis C virus (HCV) reinfection after orthotopic liver transplantation (OLT) by fibroblast growth factor-2 (FGF2) and other non-interferon mediators in the context of liver cirrhosis or hepatocellular carcinoma (HCC). Van et al. speculate that sera from post-OLT patients contained one or more factor(s) that enhance HCV infectivity (page 1017, right column, 2^{nd} paragraph). However, when characterizing the sera, Van et al. found that the change in individual mediators was highly heterogeneous between patients and the change did not reach statistical significance for any of the mediators and they could not find any clear pattern of mediators seen in those individuals whose post-OLT sera enhanced HCV *in vitro* infectivity (page 1017, right column, 3^{rd} paragraph). By randomly screening different sera components, Van et al. found that FGF2 enhanced viral replication and new particle production in about half of individuals and may thus promote reinfection, including severe forms cholestatic hepatitis.

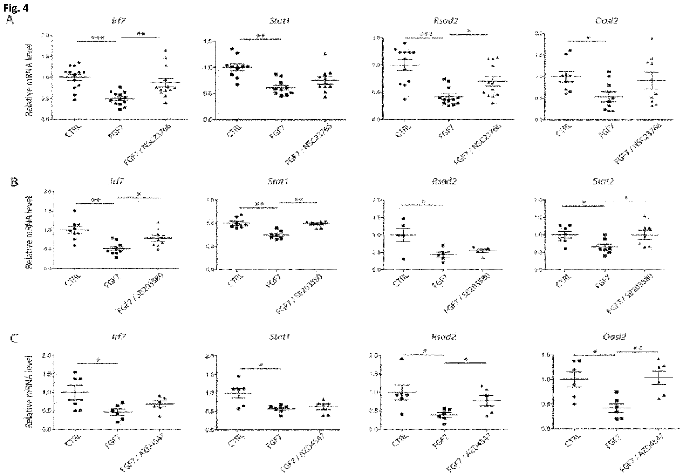

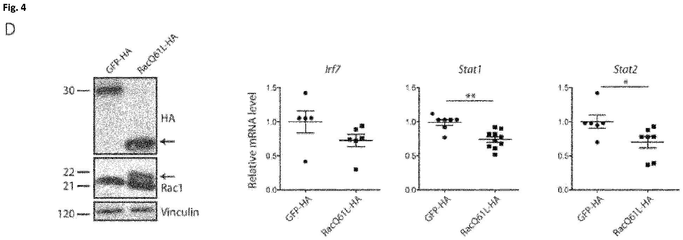

Van et al. allege that FGF2 is dependent on signaling through FGF receptor (FGFR) 3, which is not present in all tissues. In this regard, Van et al. find that other FGF2-related mediators, e.g. FGF1, do not enhance HCV (see Fig. 4 of Van et al.) in hepatic cells. This is surprising, since FGF1 binds to all FGFRs (Zhang et al., J. Biol. Chem. 2006, 281, 15694-15700), and the lack of activity of FGF1 in this experiment may be related to the overall lower biological activity of FGF1 compared to FGF2 or to the use of an insufficiently potent preparation. Importantly, hepatocytes bear a distinct FGF receptor expression pattern and it cannot be predicted whether the signaling in hepatocytes with regard to FGF and its receptor target and anti-HCV effects could be transferred to any other tissues or to any other viruses. In addition to FGFR3, hepatocytes also express high levels of FGFR4 and lower levels of FGFR2 and FGFR1, and it remains to be determined if inhibitors of other FGFRs have a similar effect and if a pan-FGFR kinase inhibitor could be even more potent compared to an FGFR3 inhibitor. In particular, Van et al. are silent on the mechanism that could be involved in FGF2's influence on HCV in liver cells. It is unknown which (if any) cellular proteins downstream of the FGF receptor are involved and responsible for the observations made in Van et al.

It is the objective of the present invention to provide new means for the treatment of viral infections.

In a first aspect, the above objective is solved by a compound for inhibiting (i) FGFR kinase activity and/or (ii) a component of the FGFR kinase signaling pathway for use in the treatment of viral infections.

It was surprisingly found that FGF signaling dramatically increases viral replication by blocking the transcription of classical interferon regulated genes. Furthermore, it was found that loss of FGFR kinase activity or its downstream targets significantly reduces viral replication also in the presence of e.g. FGF7, the classical ligand of FGFR2b (see Example 5 below). It was also found that the antiviral effect of FGFR downregulation/kinase inhibition is due to a strong induction of various interferon response genes, which inhibit different stages of the viral life cycle and is suitable for use in the treatment of viral diseases.

The advantage of FGFR inhibitors, e.g. kinase inhibitors, ligand traps or neutralizing antibodies, is that they are in or have already gone through clinical trials for cancer prevention and have been found to be well-tolerated even upon long-term applications. Therefore, most of the compounds for use in the present invention are generally more efficient, more widely applicable and have less side effects compared to currently used virostatic medicaments.

Also, the compound for inhibiting (i) FGFR kinase activity and/or (ii) a component of the FGFR kinase signaling pathway can be for use in the treatment of viral infections, wherein the viral infection is not an HCV infection and/or the infection is not a hepatocyte infection.

By way of example, it was found that various interferon target genes are under direct control of FGFs (see Examples 2 and 3 below) and these FGFs were identified as efficient inhibitors of interferon signaling through an FGFR signaling pathway involving Rac1 and p38 (see Example 4 below). Without wishing to be bound by theory, these results demonstrate the relevance of FGFR kinase pathways, for example the FGFR kinase-Rac1 pathway, for viral replication and, thus, identify FGFs as novel targets for antiviral therapies.

As used herein, the term "treatment" refers to both, prophylactic and/or therapeutic treatment unless the type of treatment is specified as prophylactic or therapeutic.

The term "inhibiting" in the context of inhibiting FGFR kinase activity and/or inhibiting a component of the FGFR kinase signaling pathway is understood to include at least partial reduction, preferably complete loss of the inherent biological function of said FGFR receptor or FGFR pathway component, including kinases and other proteins. In the context of the present invention, the reduction in biological function/activity of the FGFR kinase and/or FGFR pathway component must be to such an extent that a virus infection is significantly affected by the said reduction.

In a preferred embodiment, the composition for use according to the present invention is a composition, wherein the compound (i) for inhibiting FGFR kinase activity is selected from the group consisting of
(a) FGFR kinase inhibitors: AZD4547, Ponatinib, Dovitinib, Nintedanib, Lenvatinib, Lucitanib, Brivanib, ENMD-2076, BGJ398, FGF401, Lucitanib, PD173074, SU5402, SSR128129E, ARQ 087, LY2874455, Debio 1347, TAS-120, Erdafitinib, Nintedanib and Orantinib; FGFR ligand traps: FP1039; and FGFR neutralizing antibodies: IMC-A1, PRO-001, R3Mab, FPA144, and MGFR1877S;
(b) preferably AZD4547, BGJ398, LY2874455, Debio 1347, TAS-120, Erdafitinib ,FPA144 and FP1039;
(c) more preferably AZD4547 and BGJ398; and
(d) a physiologically acceptable salt of (a), (b) and (c).

The present invention includes physiologically acceptable salts or solvates of the compounds for use in the present invention. A "physiologically acceptable salt" refers to any physiologically acceptable salt or solvate which, upon administration to a patient, is capable of providing (directly or indirectly) (a) a compound for use according to the present invention, or (b) a pharmacologically active metabolite or pharmacologically active residue thereof. A pharmacologically active metabolite shall be understood to mean any compound being metabolized enzymatically or chemically to result in a compound for use in the present invention.

Physiologically acceptable salts include those derived from physiologically acceptable inorganic and organic acids and bases. Examples of suitable acids include, but are not limited to hydrochloric, hydrobromic, sulphuric, nitric, perchloric, fumaric, maleic, phosphoric, glycolic, lactic, salicylic, succinic, toluene-*p*-sulfuric, tartaric, acetic, citric, methanesulfonic, formic, benzoic, malonic, naphthalene-2-sulfuric and benzenesulfonic acids. Other acids, such as oxalic acid, while not themselves physiologically acceptable, may be employed in the preparation of salts useful as intermediates in obtaining the compounds and their physiologically acceptable acid addition salts. Salts derived from appropriate bases include alkali metals, preferably lithium, sodium, potassium or cesium; alkaline earth metals, preferably magnesium or calcium; ammonium, N-(C₁-C₄alkyl)₄⁺, manganese, iron, nickel, copper, zinc or aluminum salts.

In a further preferred embodiment, the composition for use according to the present invention is a composition, wherein the compound for (ii) inhibiting a component of the FGFR kinase signaling pathway is selected from the group consisting of
(a) RAC1-inhibiting compounds, preferably selected from the group consisting of NSC23766, EHop-016, Azathioprine, EHT 1864;
(b) p38 MAPK-inhibiting compounds, preferably selected from the group consisting of SB203580, VX-702, VX-745, Pamapimod, losmapimod, Dilmapimod, Doramapimod, BMS-582949, ARRY-797, PH797804, SCIO-469, SD-0006, AMG-548, LY2228820, SB239063, Skepinone L, SB202190 and TAK715; and
(c) a physiologically acceptable salt of (a) and (b).

For ease of reading, the following table provides for alternative and IUPAC names as well as CAS numbers for the compounds for use in the present invention. Of course, antibodies do not have CAS numbers and often do not feature alternative names.

| **Compound** | **Alternative name** | **Company** | **IUPAC name** | **CAS number** |
|---|---|---|---|---|
| AZD4547 | | AstraZeneca UK Ltd. | N-[5-[2-(3,5-dimethoxyphenyl)ethyl]-1H-pyrazol-3-yl]-4-[(3R,5S)-3,5-dimethylpiperazin-1-yl]benzamide | 1035270 -39-3 |
| Ponatinib | AP24534 | Ariad Pharmaceuticals, Inc., USA | 3-(2-imidazo[1,2-b]pyridazin-3-ylethynyl)-4-methyl-N-[4-[(4-methylpiperazin-1-yl)methyl]-3-(trifluoromethyl)phenyl]benzamide | 943319-70-8 |
| Dovitinib | TKI258 | Novartis AG, Switzerland | (3Z)-4-amino-5-fluoro-3-[5-(4-methylpiperazin-1-yl)-1,3-dihydrobenzimidazol-2-ylidene]quinolin-2-one | 852433-84-2 |
| Nintedanib | BIBF1120 | Boehringer Ingelheim AG & Co. KG, Germany | methyl (3Z)-3-[[4-[methyl-[2-(4-methylpiperazin-1-yl)acetyl]amino]anilino]-phenylmethylidene]-2-oxo-1H-indole-6-carboxylate | 656247-17-5 |
| Lenvatinib | E7080 | Eisai Co. Ltd., Japan | 4-[3-chloro-4-(cyclopropylcarbamoylamino)phenoxy]-7-methoxyquinoline-6-carboxamide | 417716-92-8 |
| Lucitanib | E3810 | Clovis Oncology, Inc., USA | 6-[7-[(1-aminocyclopropyl)methoxy]-6-methoxyquinolin-4-yl]oxy-N-methylnaphthalene-1-carboxamide | 1058137 -23-7 |
| Brivanib | BMS540215 | Bristol-Myers Squibb Co., USA | (2R)-1-[4-[(4-fluoro-2-methyl-1H-indol-5-yl)oxy]-5-methylpyrrolo[2,1-f][1,2,4]triazin-6-yl]oxypropan-2-ol | 649735-46-6 |
| ENMD-2076 | | CASI Pharmaceuticals Inc., USA | 6-(4-methylpiperazin-1-yl)-N-(5-methyl-1H-pyrazol-3-yl)-2-[(E)-2-phenylethenyl]pyrimidin-4-amine | 934353-76-1 |
| BGJ398 | | Novartis AG, Switzerland | 3-(2,6-dichloro-3,5-dimethoxyphenyl)-1-[6-[4-(4-ethylpiperazin-1-yl)anilino]pyrimidin-4-yl]-1-methylurea | 872511-34-7 |
| FGF401 | | Novartis AG, Switzerland | | - |
| Lucitanib | E-3810 | Eisai Co. Ltd., Japan | 6-[7-[(1-aminocyclopropyl)methoxy]-6-methoxyquinolin-4-yl]oxy-N-methylnaphthalene-1-carboxamide | 1058137 -23-7 |
| PD173074 | | Pfizer, Inc., USA | 1-tert-butyl-3-[2-[4-(diethylamino)butylamino]-6-(3,5-dimethoxyphenyl)pyrido[2,3-d]pyrimidin-7-yl]urea | 219580-11-7 |
| SU5402 | | SUGEN, USA | 3-[4-methyl-2-[(Z)-(2-oxo-1H-indol-3-ylidene)methyl]-1H-pyrrol-3-yl]propanoic acid | 215543-92-3 |
| SSR128129E | | Sanofi SA, France | sodium;2-amino-5-(1-methoxy-2-methylindolizine-3-carbonyl)benzoate | 848318-25-2 |
| ARQ 087 | | ArQule, USA | (6R)-6-(2-fluorophenyl)-5,6-dihydro-N-[3-[2-[(2-methoxyethyl)amino]ethyl]phenyl]-Benzo[h]quinazolin-2-amine | 1234356 -69-4 |
| LY2874455 | | Eli Lilly and Co., USA | 2-[4-[(E)-2-[5-[(1R)-1-(3,5-dichloropyridin-4-yl)ethoxy]-1H-indazol-3-yl]ethenyl]pyrazol-1-yl]ethanol | 1254473 -64-7 |
| Debio 1347 | CH-5183284 | Debiopharm SA, Switzerland | [5-amino-1-(2-methyl-3H-benzimidazol-5-yl)pyrazol-4-yl]-(1H-indol-2-yl)methanone | 1265229 -25-1 |
| TAS-120 | | Taiho Pharmaceutical Co., Ltd., Japan | | 1448169 -71-8 |
| Erdafitinib | JNJ4275649 3 | Astex Therapeutics, UK | N1-(3,5-dimethoxyphenyl)-N2-isopropyl-N1-(3-(1-methyl-1H-pyrazol-4-yl)quinoxalin-6-yl)ethane-1,2-diamine | 1346242 -81-6 |
| Nintedanib | BIBF1120 | Boehringer Ingelheim AG & Co. KG, Germany | Methyl (3Z)-3-{[(4-{methyl[(4-methylpiperazin-1-yl)acetyl]amino}phenyl)amino](phenyl) methylidene}-2-oxo-2,3-dihydro-1H-indole-6-carboxylate | 656247-17-5 |
| Orantinib | TSU68 / SU6668 | Taiho Pharmaceutical Co., Ltd., Japan | 3-[2,4-dimethyl-5-[(Z)-(2-oxo-1H-indol-3-ylidene)methyl]-1H-pyrrol-3-yl]propanoic acid | 252916-29-3 |
| FP1039 | GSK3052230 | Five Prime Therapeutics Inc., USA | | |
| IMC-A1 | | ImClone Systems LLC, USA | | |
| PRO-001 | | ProChon Biotech Ltd., USA | | |
| R3Mab | | Genentech, USA | | |
| FPA144 | | Five Prime Therapeutics Inc., USA | | |
| MGFR1877S | RG744 | Genentech, USA | | |
| NSC23766 | | Calbiochem, Merck KGaA, Germany | 6-N-[2-[5-(diethylamino)pentan-2-ylamino]-6-methylpyrimidin-4-yl]-2-methylquinoline-4,6-diamine | 1177865 -17-6 |
| EHop-016 | | - | N4-(9-Ethyl-9H-carbazol-3-yl)-N2-(3-morpholinopropyl)pyrimidine-2,4-diamine | 1380432 -32-5 |
| Azathioprine | Imuran | - | 6-(3-methyl-5-nitroimidazol-4-yl)sulfanyl-7H-purine | 446-86-6 |
| EHT 1864 | | - | 2-(morpholin-4-ylmethyl)-5-[5-[7-(trifluoromethyl)quinolin-4-yl]sulfanylpentoxy]pyran-4-one;dihydrochloride | 754240-09-0 |
| SB203580 | | - | 4-[4-(4-fluorophenyl)-2-(4-methylsulfinylphenyl)-1H-imidazol-5-yl]pyridine | 152121-47-6 |
| VX-702 | KVK702 | Vertex Pharmaceuticals, USA | 6-(N-carbamoyl-2,6-difluoroanilino)-2-(2,4-difluorophenyl)pyridine-3-carboxamide | 479543-46-9 |
| VX-745 | | Vertex Pharmaceuticals, USA | 5-(2,6-dichlorophenyl)-2-(2,4-difluorophenyl)sulfanylpyrimido[1,6-b]pyridazin-6-one | 209410-46-8 |
| Pamapimod | RO4402257 | Roche Pharma AG, Switzerland | 6-(2,4-difluorophenoxy)-2-(1,5-dihydroxypentan-3-ylamino)-8-methylpyrido[2,3-d]pyrimidin-7-one | 449811-01-2 |
| Losmapimod | GW856553 | GlaxoSmithKline LLC, UK | 6-[5-(cyclopropylcarbamoyl)-3-fluoro-2-methylphenyl]-N-(2,2-dimethylpropyl)pyridine-3-carboxamide | 585543-15-3 |
| Dilmapimod | SB681323 | GlaxoSmithKline LLC, UK | 8-(2,6-difluorophenyl)-2-((1,3-dihydroxypropan-2-yl)amino)-4-(4-fluoro-2-methylphenyl)pyrido[2,3-d]pyrimidin-7(8H)-one | 444606-18-2 |
| Doramapimod | BIRB 796 | Boehringer Ingelheim AG & Co. KG, Germany | 1-[5-tert-butyl-2-(4-methylphenyl)pyrazol-3-yl]-3-[4-(2-morpholin-4-ylethoxy)naphthalen-1-yl]urea | 285983-48-4 |
| BMS-582949 | PS540446 | Bristol-Myers Squibb Co., USA | 4-[5-(cyclopropylcarbamoyl)-2-methylanilino]-5-methyl-N-propylpyrrolo[2,1-f][1,2,4]triazine-6-carboxamide | 912806-16-7 |
| ARRY-797 | ARRY37179 7 | Array BioPharma Inc., USA | 5-(2,4-difluorophenoxy)-N-[2-(dimethylamino)ethyl]-1-(2-methylpropyl)indazole-6-carboxamide | - |
| PH797804 | | Pfizer, Inc., USA | 3-[3-bromo-4-[(2,4-difluorophenyl)methoxy]-6-methyl-2-oxopyridin-1-yl]-N,4-dimethylbenzamide | 1358027 -80-1 |
| SCIO-469 | | Scios, Inc., USA | bismuth(2+);2-tert-butylbenzene-1,4-diol;2-oxidobenzoate;hydrate | 309913-83-5 |
| SD-0006 | SD06 | GlaxoSmithKline LLC, UK | 1-[4-[3-(4-chlorophenyl)-4-pyrimidin-4-yl-1H-pyrazol-5-yl]piperidin-1-yl]-2-hydroxyethanone | 271576-80-8 |
| AMG-548 | | Amgen Inc., USA | 2-[[(2S)-2-Amino-3-phenylpropyl]amino]-3-methyl-5-(2-naphthalenyl)-6-(4-pyridinyl)-4(3H)-pyrimidinone | 864249-60-5 |
| LY2228820 | | Eli Lilly and Co., USA | 5-[2-tert-butyl-4-(4-fluorophenyl)-1H-imidazol-5-yl]-3-(2,2-dimethylpropyl)imidazo[4,5-b]pyridin-2-amine;methanesulfonic acid | 862507-23-1 |
| SB239063 | | GlaxoSmithKline LLC, UK | 4-[4-(4-fluorophenyl)-5-(2-methoxypyrimidin-4-yl)imidazol-1-yl]cyclohexan-1-ol | 193551-21-2 |
| Skepinone L | | - | 3-(2,4-difluoroanilino)-9-[(2R)-2,3-dihydroxypropoxy]-5,6-dihydrodibenzo[3,1-[7]annulen-11-one | 1221485 -83-1 |
| SB202190 | | - | 4-[4-(4-fluorophenyl)-5-pyridin-4-yl-1,3-dihydroimidazol-2-ylidene]cyclohexa-2,5-dien-1-one | 152121-30-7 |
| TAK715 | | Takeda KK, Japan | N-[4-[2-ethyl-4-(3-methylphenyl)-1,3-thiazol-5-yl]pyridin-2-yl]benzamide | 303162-79-0 |

Preferably, the viral infection to be treated by the compounds for use in the present invention are selected from the group consisting of Herpes simplex virus 1 and/or 2, Human Papilloma viruses, Ebola virus, Marburg virus, Venezuelan Equine encephalitis virus, Chikungunya virus, Easter Equine Encephalitis virus, Western Equine Encephalitis virus, Monkey Pox virus, Corona virus, Respiratory Syncytial virus, Adenovirus, Human Rhinovirus, Influenza virus, HIV, HCV, Norovirus, Saporovirus, cytomegalovirus (CMV), Dengue virus, West Nile virus, Yellow fever virus, Zika Virus, HSV1, HSV2, HIV1, HIV2, HCV, SARS virus or MARS virus and HBV.

In another preferred embodiment, the composition for use according to the present invention is a composition, wherein
(a) the compound is selected from the group consisting of AZD4547, BGJ398, FP1039, FPA144 and physiologically acceptable salts thereof; and
(b) the viral infection is caused by a virus selected from the group consisting of Herpes simplex virus 1 and/or 2, Human Papilloma viruses, Ebola virus, Marburg virus, Venezuelan Equine encephalitis virus, Chikungunya virus, Easter Equine Encephalitis virus, Western Equine Encephalitis virus, Monkey Pox virus, Corona virus, Respiratory Syncytial virus, Adenovirus, Human Rhinovirus, Influenza virus, HIV, HCV, Norovirus, Saporovirus, cytomegalovirus (CMV), Dengue virus, West Nile virus, Yellow fever virus, Zika Virus, and HBV.

In a further preferred embodiment, the composition for use according to the present invention is a composition, wherein the compound is selected from the group consisting of **FGFR kinase inhibitors:** AZD4547, Ponatinib, Dovitinib, Nintedanib, Lenvatinib, Lucitanib, Brivanib, ENMD-2076, BGJ398, FGF401, Lucitanib, PD173074, SU5402, SSR128129E, ARQ 087, LY2874455, Debio 1347, TAS-120, Erdafitinib, Nintedanib, and Orantinib; **FGFR ligand traps:** FP1039; and **FGFR neutralizing antibodies:** IMC-A1, PRO-001, R3Mab, FPA144 and MGFR1877S; and physiologically acceptable salts thereof; and the viral infection is caused by a virus selected from the group consisting of Dengue virus, HSV1, HSV2, HIV1, HIV2, HCV, Zika Virus, Influenza virus, SARS virus or MARS virus.

In another preferred embodiment, the composition for use according to the present invention is a composition, wherein the compound is selected from the group consisting of AZD4547, BGJ398 and physiologically acceptable salts thereof; and the viral infection is caused by a virus selected from the group consisting of HSV1, HSV2 and Zika Virus.

In a more preferred embodiment the compound for use according to the present invention is
- (i) a compound for inhibiting FGFR 1, FGFR 2, and/or FGFR 3 kinase activity, or a compound for inhibiting FGFR 1, FGFR 2, and/or FGFR 3 kinase signaling, for the treatment of a viral disease in epithelial cells of the skin (keratinocytes), preferably an HSV1 or HSV2 infection in keratinocytes, for example, a compound selected from the group consisting of AZD4547, Ponatinib, Dovitinib, Nintedanib, Lenvatinib, Lucitanib, Brivanib, ENMD-2076, BGJ398, FGF401, Lucitanib, PD173074, SU5402, SSR128129E, ARQ 087, LY2874455, Debio 1347, TAS-120, Erdafitinib, Nintedanib, Orantinib and FPA144;
- preferably (ii) an FGFR ligand trap binding ligands of FGFR2b for the treatment of a viral disease affecting epithelial cells of the skin (keratinocytes), preferably an HSV1 or HSV2 infection of keratinocytes, or for the treatment of a viral disease affecting the lung, preferably an influenza virus infection of the lung, for example, a compound selected from the group consisting of AZD4547, Ponatinib, Dovitinib, Nintedanib, Lenvatinib, Lucitanib, Brivanib, ENMD-2076, BGJ398, FGF401, Lucitanib, PD173074, SU5402, SSR128129E, ARQ 087, LY2874455, Debio 1347, TAS-120, FP1039, Erdafitinib, Nintedanib, Orantinib and FPA144;
- more preferably (iii) a compound for inhibiting FGFR 1, FGFR 2, FGFR 3 and/or FGFR 4 kinase activity, or a compound for inhibiting FGFR 1, FGFR 2, FGFR 3 and/or FGFR 4 kinase signaling for the treatment of a viral disease affecting T cells, preferably an HIV infection of T cells, for example, a compound selected from the group consisting of AZD4547, Ponatinib, Dovitinib, Nintedanib, Lenvatinib, Lucitanib, Brivanib, ENMD-2076, BGJ398, FGF401, Lucitanib, PD173074, SU5402, SSR128129E, ARQ 087, LY2874455, Debio 1347, TAS-120, Erdafitinib, Nintedanib, FP1039 and Orantinib; or
- most preferably (iv) a compound for inhibiting FGFR 1, FGFR 2, FGFR 3, and/or FGFR 4 kinase activity, or a compound for inhibiting FGFR 1, FGFR 2, FGFR 3 and/or FGFR 4 kinase signaling for the treatment of a viral disease in hepatocytes, preferably an HCV or HBV infection in hepatocytes, for example, a compound selected from the group consisting of AZD4547, Ponatinib, Dovitinib, Nintedanib, Lenvatinib, Lucitanib, Brivanib, ENMD-2076, BGJ398, FGF401, Lucitanib, PD173074, SU5402, SSR128129E, ARQ 087, LY2874455, Debio 1347, TAS-120, FP1039, Erdafitinib, Nintedanib, and Orantinib.

A further preferred embodiment relates to a compound, wherein the compound is a compound for use in the present invention for inhibiting at least FGFR 1 and FGFR 2 kinase activity, or a compound for inhibiting at least FGFR 1 and FGFR 2 kinase signaling, preferably, FP1039, FPA144, AZD4547 or BGJ398, for use in the treatment of a viral disease in keratinocytes, preferably an HSV1 or HSV2 infection in keratinocytes.

In a further preferred embodiment, the composition for use according to the present invention is a pharmaceutical composition comprising at least one compound for use according to the present invention and optionally further physiologically acceptable excipients as defined above.

The compounds for use in the present invention may be administered alone or in combination with adjuvants that enhance stability, facilitate administration of pharmaceutical compositions containing them, provide increased dissolution or dispersion, increase inhibitory activity, provide adjunct therapy, and the like, including other active ingredients. The above described compounds may be physically combined with other adjuvants into a single pharmaceutical composition. Reference in this regard may be made to Cappola et al.: U.S. patent application no. 09/902,822, PCT/US 01/21860 und US provisional application no. 60/313,527, each incorporated by reference herein in their entirety. The optimum percentage (w/w) of a compound or composition of the invention may vary and is within the purview of those skilled in the art. Alternatively, the compounds may be administered separately (either serially or in parallel). Separate dosing allows for greater flexibility in the dosing regimen.

As mentioned above, dosage forms of the compounds for use in the present invention include pharmaceutically acceptable carriers and adjuvants known to those of ordinary skill in the art. These carriers and adjuvants include, for example, ion exchangers, alumina, aluminium stearate, lecithin, serum proteins, buffer substances, water, salts or electrolytes and cellulose-based substances. Preferred dosage forms include, tablet, capsule, caplet, liquid, solution, suspension, emulsion, lozenges, syrup, reconstitutable powder, granule, suppository and transdermal patch. Methods for preparing such dosage forms are known (see, for example, H. C. Ansel and N. G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient. In some embodiments, dosage levels range from about 5 mg - 500 mg/dose for a 70 kg patient. Although one dose per day may be sufficient, up to 5 doses per day may be given. For oral doses, up to 2000 mg/day may be required. Reference in this regard may also be made to US provisional application no. 60/339,249. As the skilled artisan will appreciate, lower or higher doses may be required depending on particular factors. For instance, specific doses and treatment regimens will depend on factors such as the patient's general health profile, the severity and course of the patient's disorder or disposition thereto, and the judgment of the treating physician. For example, the compounds of the present invention can be administered the same way as other virostatic medicaments.

Compounds for use in the present invention may be formulated into capsules the same way other virostatic medicaments are formulated. Each capsule may contain 25 to 500, preferably 150 to 300, more preferably 200 to 250 mg of a compound of the invention. For example, non-medicinal ingredients in capsules for the compounds of the present invention are - capsule shell: D&C yellow No. 10, FD&C blue No. 1, FD&C red No. 3, FD&C yellow No. 6, gelatin and titanium dioxide. Bottles of 100. (see also Martindale: the complete drug reference, 34th Edition, 2005, Pharmaceutical Press, p 612.)

In a further preferred embodiment, the pharmaceutical composition for use according to the present invention is for topical, oral, intravenous, intranasal, or rectal administration.

Routes of administration also include, but are not limited to intraperitoneally, intramuscularly, subcutaneously, intrasynovially, by infusion, sublingually, transdermally, or by inhalation. The preferred modes of administration are topical, oral, intravenous, intranasal, or rectal administration.

In another preferred embodiment, the pharmaceutical composition for use according to the present invention is a composition, wherein the at least one compound is selected from the group consisting of AZD4547, BGJ398 and physiologically acceptable salts thereof; the composition is for oral, intranasal, intravenous, intramuscular, intradermal, subcutaneous, intraperitoneal or topical administration, preferably topical administration; and the composition is for use in the treatment of HSV-1 inventions.

In a further preferred embodiment, the compounds or compositions defined above are used for the prophylactic or therapeutic treatment of an HSV 1 infection in keratinocytes, and the compound or composition is preferably for topical administration.

In another preferred embodiment, the compounds or compositions as defined above are for use in the prophylactic or therapeutic treatment of a viral infection in a human or animal, preferably a human, mammal or bird, more preferably a human.

Another aspect of the present invention is directed to a method for the therapeutic or prophylactic treatment of a viral disease, preferably one or more of the viral diseases listed above, comprising the steps of
(a) providing a compound or a composition as defined above; and
(b) administering the compound or composition of (a) to the subject in need thereof in a pharmaceutically effective amount, preferably by oral, intranasal, intravenous, intramuscular, intradermal, subcutaneous, intraperitoneal or topical administration, more preferably by topical administration.

Preferably the subject for treatment is selected from the group consisting of a human and an animal, preferably a human, mammal and bird, more preferably a human.

Also, the present invention relates to the use of a compound for inhibiting (i) FGFR kinase activity or (ii) a component of the FGFR kinase signaling pathway as described above in the manufacture of a medicament for the treatment of a viral infection, preferably a viral infection as defined above.

### Figures

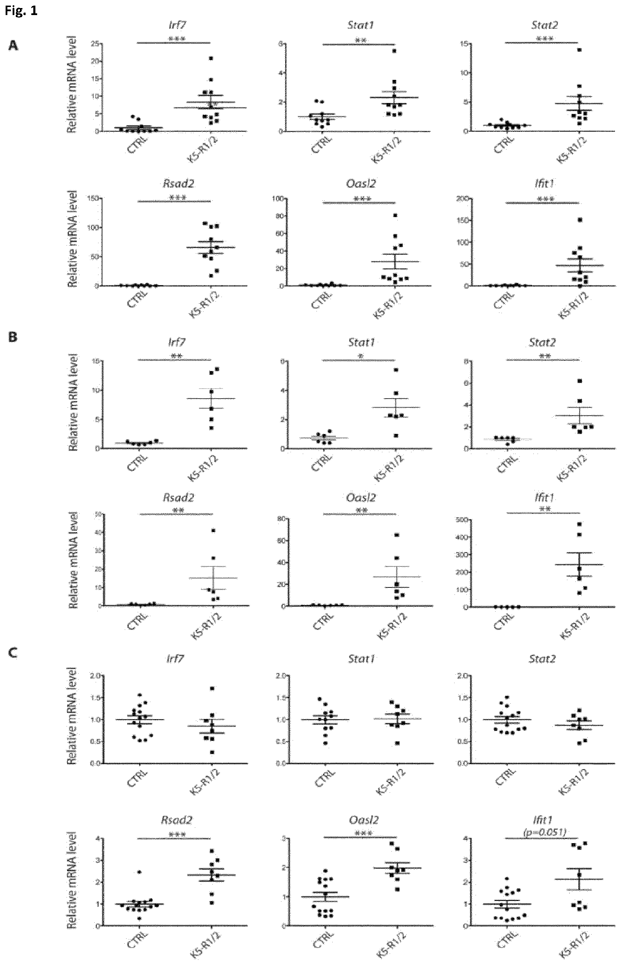

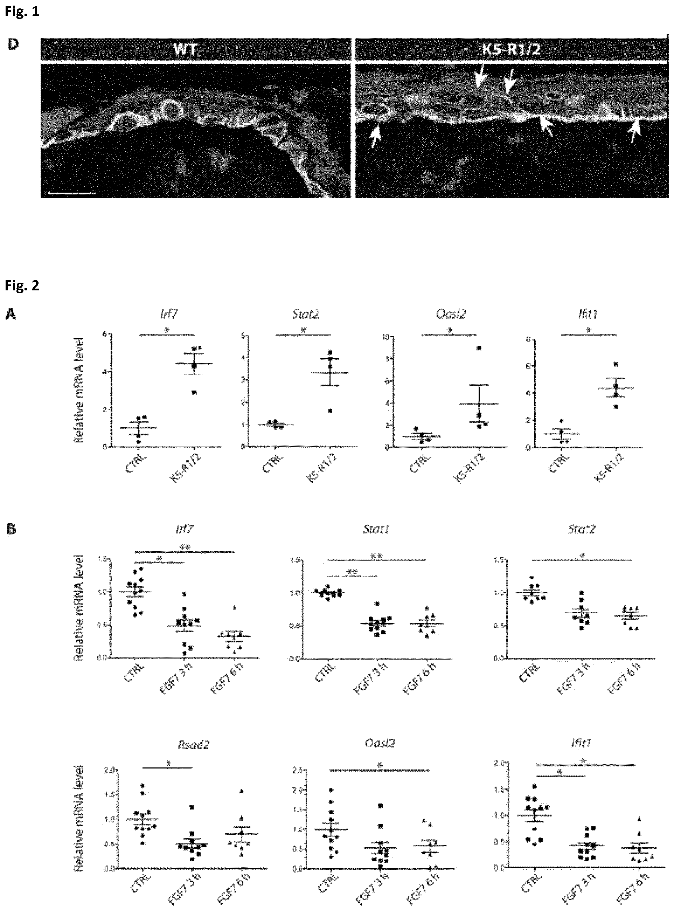

**Fig. 1** shows enhanced expression of Interferon Stimulated Genes (ISGs) in the epidermis of FGFR1/R2-deficient mice. **A-C:** qRT-PCR analysis of *Irf7, Stat1, Stat2, Rsad2, Oasl2* and *Ifit1* relative to *Rps29* using epidermal RNA of FGFR1/R2 knockout (K5-R1/R2) and control (CTRL) mice at the age of 3 months **(A),** 9 days **(B)** or 5 days **(C).** Expression levels in WT mice were arbitrarily set to 1. **D)** Confocal microscopy images of epidermal sheets from back skin of 3 month-old CTRL and K5-R1/R2 mice stained with antibodies against IRF7 and K14; counterstained with DAPI. Arrows denote increased expression and nuclear localization of IRF7 in K5-R1/R2 mice. Magnification bar: 20 µm. Data are representative of two experiments. Bars indicate mean ± SD. **p* ≤ 0.05, ***p* ≤ 0.01, ****p* ≤ 0.001.

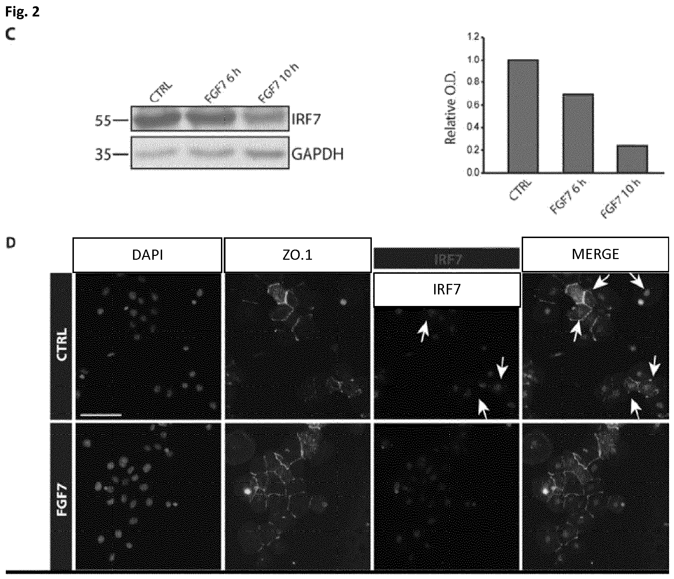

**Fig. 2** shows FGF signaling modulates ISGs expression in cultured keratinocytes. **A)** Primary keratinocytes from WT and K5-R1/R2 mice were analyzed for *Irf7, Stat2, Oasl2* and *Ifit1* expression relative to *Rps29* by qRT-PCR. Expression levels in WT mice were arbitrarily set to 1. **B-D)** Primary keratinocytes from WT mice were starved overnight in serum-free medium and subsequently treated for 3, 6 or 10 h with FGF7 (10 ng/ml) or vehicle. **(B)** RNA from the treated cells was analyzed by qRT-PCR for the expression of the indicated ISGs relative to *Rps29.* Expression levels in vehicle-treated (CTRL) cells were arbitrarily set to 1. **(C)** Protein lysates were analyzed for expression of IRF7 by western blotting. Probing of the membrane with an antibody against GAPDH was used as a loading control. Quantification of the band intensities is shown in the bar graph. **(D)** Cells were stained with anti-IRF7 and, to visualize cell junctions, with anti-ZO.1 antibodies. Cell nuclei were stained with DAPI. Arrows indicate nuclear localization of IRF7 in CTRL samples. Images were obtained with standard wide field microscopy. Magnification bar: 50 µm. Data are representative of three experiments. Bars indicate mean ± SD. **p* ≤ 0.05, ***p* ≤ 0.01.

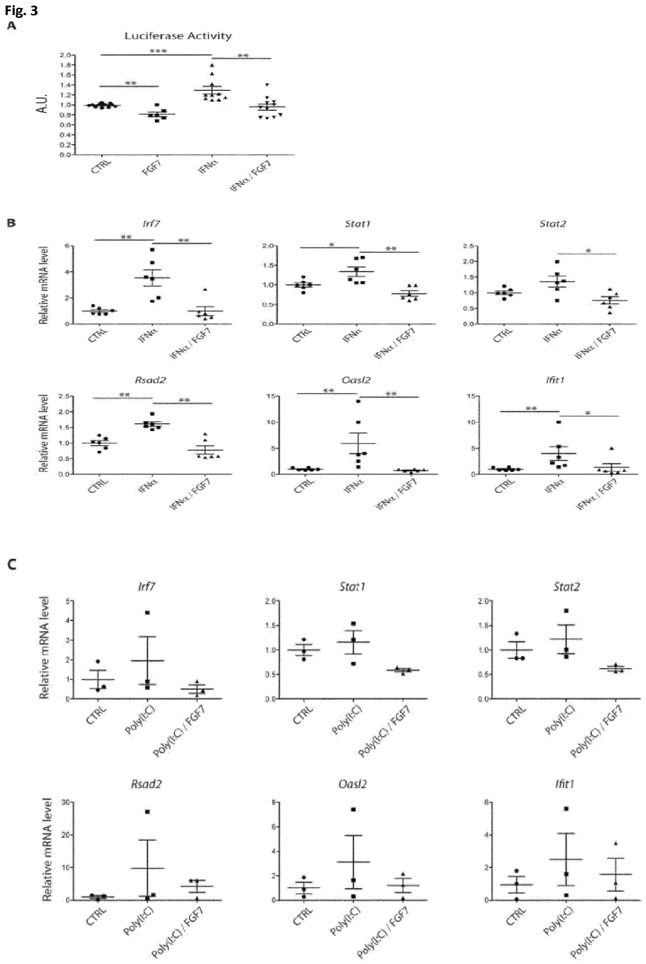

**Fig. 3** shows that FGF7 suppresses IFNα- and Poly(I:C)-mediated up-regulation of ISGs in keratinocytes at the transcriptional level. **A)** Immortalized mouse keratinocytes were co-transfected with TK-Renilla and pGL4.45[*luc2P*/ISRE/Hygro] plasmids, starved in serum-free medium and then treated with FGF7 (10 ng/ml) and/or IFNα (1000 U/ml) for 12 h. After cell lysis, firely luciferase activity was normalized to Renilla luciferase activity (transfection control). The value in vehicle-treated cells (CTRL) was arbitrarily set to 1. **B, C)** Serum-starved primary keratinocytes from WT mice were stimulated with IFNα alone and in combination with FGF7 **(B),** or with Poly(I:C) (5 µg/ml) alone and in combination with FGF7 **(C)** for 12 h. Cells were harvested and analyzed for ISG expression relative to *Rps29* by qRT-PCR. Expression levels in vehicle-treated (CTRL) cells were arbitrarily set to 1. Data are representative of two experiments. Bars indicate mean ± SD. **p* ≤ 0.05, ***p* ≤ 0.01, ****p* ≤ 0.001.
**Fig. 4** shows that FGF7 modulates ISG expression via Rac1 and p38. **A-C)** Serum-starved primary keratinocytes from WT mice were incubated with the Rac1 inhibitor NSC23766 (50 µM) **(A),** the p38 inhibitor SB203580 (5 µM) **(B)** or the FGFR1/2/3 kinase inhibitor AZD4547 (1 µM) **(C)** for 4 h, followed by treatment with FGF7 (10 ng/ml) for 6 h. RNA from the treated cells was used to analyze ISG expression levels by qRT-PCR relative to *Rps29.* Expression levels in vehicle-treated cells were arbitrarily set to 1. Data are representative of three experiments. **D)** Immortalized murine keratinocytes were transfected with pcDNA3.1-Rac1Q61L-HA and pcDNA3.1-EGFP-HA expression vectors, resulting in expression of recombinant proteins with an epitope tag of the influenza virus hemagglutinin (HA). After 24 h, cells were lysed and analyzed by Western blot for Rac1, HA and Vinculin (loading control) (left). Arrows indicate expression of recombinant Rac1Q61L-HA. In addition, cells were analyzed by qRT-PCR for *Irf7, Stat*1 and *Stat2* relative to *Rps29* (right). Expression levels in pcDNA3.1-GFP-HA transfected cells were arbitrarily set to 1. Data are representative of two experiments. Bars indicate mean ± SD. **p* ≤ 0.05, ***p* ≤ 0.01, ****p* ≤ 0.001.

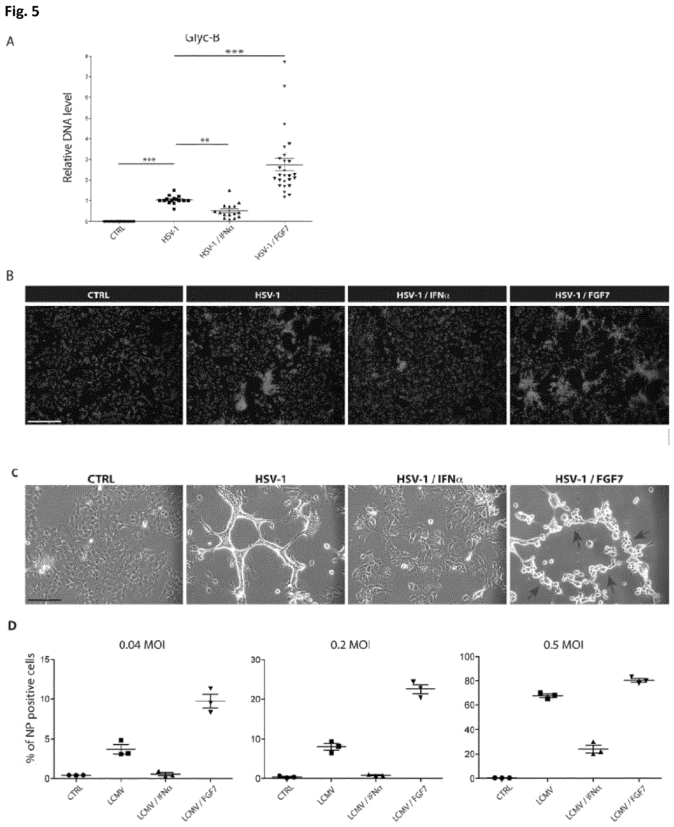

**Fig. 5** shows that FGF7 promotes HSV-1 and LCMV replication in HaCaT cells. **A-C**) After overnight serum starvation, HaCaT cells were infected with HSV-1 (MOI = 0.5) alone or in combination with FGF7 (15 ng/ml) or IFNα- (1000U/ml). **A, B)** 24 h after HSV-1 infection, cells were harvested and virus load was measured by qPCR of the HSV-1 glycoprotein B gene (Glyc-B) relative to the human β-actin gene **(A)** or visualized by HSV-1 glycoprotein D (Glyc-D) staining (red) **(B).** The Glyc-B/β-actin gene ratio in HSV-1 infected cells was arbitrarily set to 1. Cell nuclei were stained with DAPI (blue). Magnification bar: 200 µm. **C)** Transmission microscopy images of HaCaT 48 h after HSV-1 infection. Arrows indicate detached cell clusters in HSV-1-infected samples, which had been treated with FGF7. Magnification bar: 80 µm. Data are representative of four experiments. **D)** HaCaT cells were infected LCMV (MOI as indicated) in the presence or absence of FGF7 or IFNα. 24h later they were analyzed for LCMV nucleoprotein (NP) expression by flow cytometry. Bars indicate mean ± SD. **p* ≤ 0.05, ***p* ≤ 0.01, ****p* ≤ 0.001.

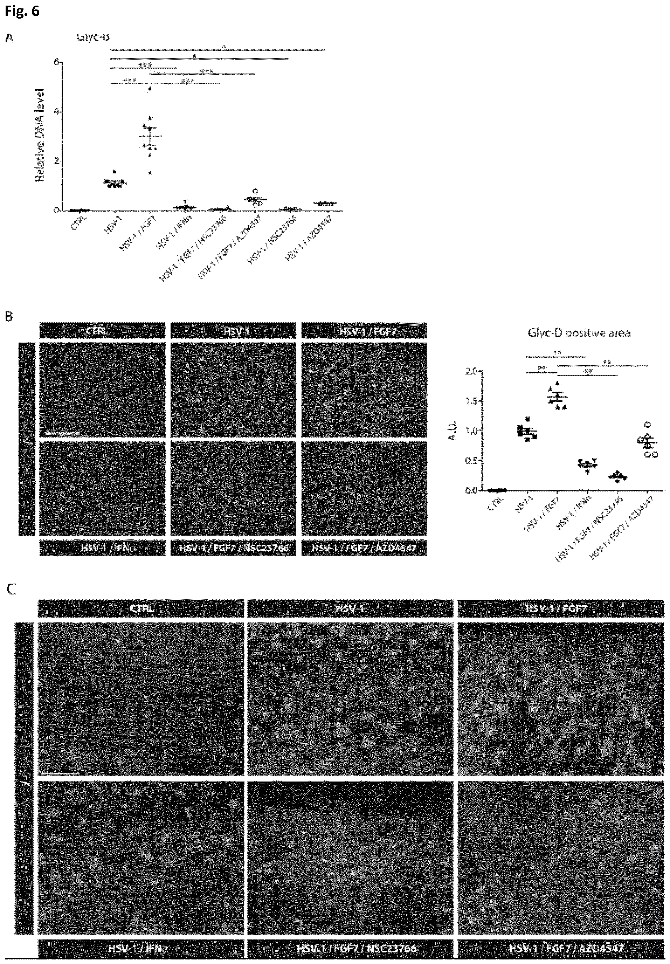

**Fig. 6** shows that FGF7 promotes HSV-1 replication *in vitro* and *ex vivo* via FGFR kinase activity and Rac1. **A, B)** Serum-starved HaCaT cells were infected with HSV-1 in the presence or absence of FGF7 (15 ng/ml), NSC23766 (50 µM) or the FGFR kinase inhibitor AZD4547 (1 µM). After 24 h, the viral load was analyzed by qPCR analysis for the gene encoding viral Glyc-B relative to the human β-actin gene **(A)** and by Glyc-D staining (red) **(B, left).** Magnification bar: 500 µm. The Glyc-D positive area was quantified using digital pixel measurements **(B, right).** The Glyc-B/β-actin gene ratio in HSV-1 infected cells was arbitrarily set to 1. Data are representative of three experiments. **C)** Isolated epidermal sheets from tail skin were infected *ex vivo* with HSV-1 (MOI = 2) in the presence or absence of IFNα, FGF7, NSC23766 or AZD4547 and stained 48 h after infection with antibodies against Glyc-D (red). Note the strong increase in infected cells after FGF treatment. Cell nuclei were stained using DAPI (blue). Magnification bar: 800 µm. Data are representative of three experiments. Bars indicate mean ± SD. **p* ≤ 0.05, ***p* ≤ 0.01, ****p* ≤ 0.001.

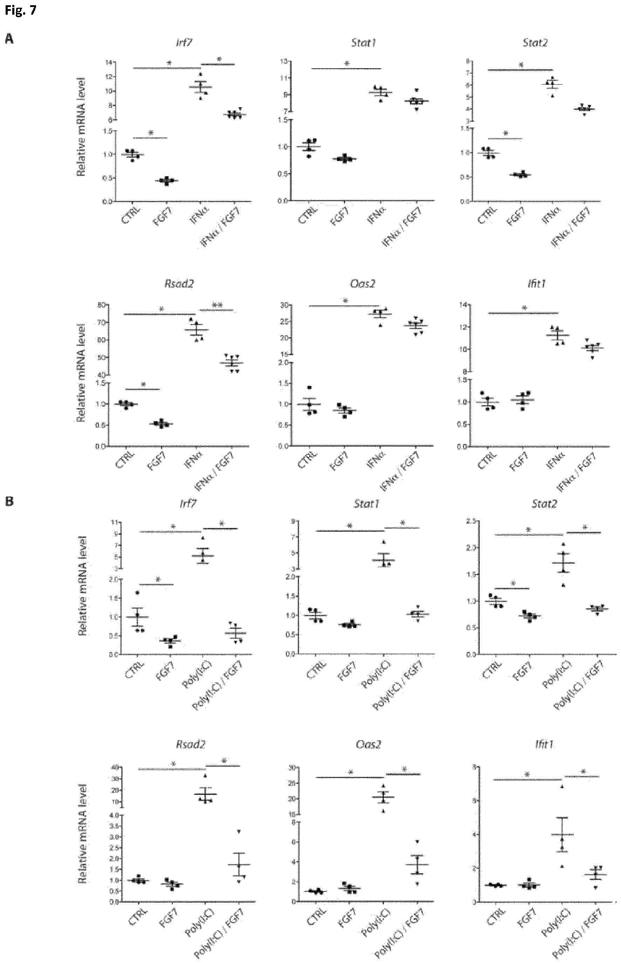

**Fig. 7** shows that FGF7 suppresses IFNα- and Poly(I:C)-mediated up-regulation of ISGs expression in HaCaT cells. **A, B)** HaCaT cells were serum-starved overnight and treated with FGF7 (15 ng/ml) and/or IFNα (1000 U/ml) **(A),** and/or Poly(I:C) (5 µg/ml) **(B)** for 12 h. Cells were harvested and analyzed for ISG expression levels by qRT-PCR relative to *RPL27.* Expression levels in vehicle-treated (CTRL) cells were arbitrarily set to 1. Data are representative of two experiments. Bars indicate mean ± SD. **p* ≤ 0.05, ***p* ≤ 0.01.

### Examples

### Example 1: Materials and methods

### Antibodies, recombinant proteins and chemical compounds

The following antibodies were used Western blotting and/or immunostaining: anti- IRF7 (sc-9083, Santa Cruz, CA), anti-glyceraldehyde 3-phosphate dehydrogenase (GAPDH) (5G4, HyTest, Turku, Finland), anti-Zona Occludens 1 (ZO.1) (339100, Invitrogen, Carlsbad, CA), anti-Keratin 14 (K14) (PRB-155P, BioLegend, San Diego, CA), anti-human influenza virus hemagglutinin (HA) (H6908, Sigma, Munich, Germany), anti-Vinculin (v4505, Sigma), anti-Rac1 (05-389, Merck-Millipore, Darmstadt, Germany), anti-HSV-1 glycoprotein D (Glyc-D) (ab27586, Abcam, Cambridge, UK), anti- LCMV nucleoprotein (clone VL-4) (kindly provided by Prof. Rolf M. Zinkernagel, University of Zurich) (Battegay et al., 1991), AF555-conjugated anti-mouse IgG (A-21422, Thermo Fisher, Waltham, MA), AF488-conjugated anti-mouse IgG (A-11001, Thermo Fisher), and AF555-conjugated anti-rabbit IgG (A-21428, Thermo Fisher). The following recombinant proteins and compounds were used: recombinant human FGF7 (100-19, PeproTech Inc., Rocky Hill, NJ), recombinant human IFNα (300-02AA, PeproTech Inc.), polyinosinic-polycytidylic acid (Poly(I:C) (tlrl-pic, InvivoGen, San Diego, CA)), Rac inhibitor NSC23766 (S8031, Selleckchem, Houston, TX), p38 MAPK inhibitor SB203580 (S1076, Selleckchem), FGFR1/2/3 inhibitor AZD4547 (S2801, Selleckchem).

### Genetically modified mice

Mice lacking Fgfr1 and Fgfr2 in keratinocytes (K5-R1/R2 mice and their control littermates) had previously been described (Yang et al., 2010). All mice were in C57BI/6 genetic background. They were housed under optimal hygiene conditions and maintained according to Swiss animal protection guidelines. The local veterinary authorities of Zurich, Switzerland, had approved all procedures with mice.

### Cell culture

Primary keratinocytes were isolated from neonatal mice as described previously (Yang et al., 2010) and cultured for 3 days in a 7:5 mixture of keratinocyte serum-free medium (Life Technologies, Carlsbad, CA) supplemented with 10 ng/ml EGF, 10-10 M cholera toxin and 100 U/ml penicillin/100 µg/ml streptomycin (Sigma) and of keratinocyte medium. Plates were coated with collagen IV (Sigma) prior to seeding of the cells. Spontaneously immortalized keratinocytes from wild-type mice had previously been described (Yang et al., 2010). For treatment of primary or immortalized mouse keratinocytes with FGF7 (10 ng/ml) and/or IFNα (1000 U/ml) and/or Poly(I:C) (5 µg/ml) they were starved overnight in keratinocyte serum-free medium, treated for the indicated time points, and harvested for RNA or protein extraction. For the treatment with NSC23766 (50 µM), SB203580 (5 µM), AZD4547 (1 µM) or vehicle (DMSO), cells were preincubated with the indicated inhibitors for 4 h at 37°C prior to treatment with FGF7 (10 ng/ml) and harvested 6 h later. The human HaCaT keratinocyte cell line (Boukamp et al., 1988) was cultured in Dulbecco's modified Eagle's Medium (DMEM) (Sigma) supplemented with 10% fetal calf serum (FCS) (Thermo Fisher). For FGF7 or IFNα treatment they were grown to sub-confluency and serum-starved overnight prior to addition of 15 ng/ml FGF7 and/or 1000 U/ml IFNα and/or Poly(I:C) (5 µg/ml) for 12 h.

### Separation of dermis from epidermis of mouse back skin

Separation of epidermis from dermis was achieved either by heat shock treatment (30 sec at 55-60°C followed by 1 min at 4°C, both in PBS), by incubation for 50-60 min at 37°C in 0.143 % dispase (17105-041, Life Technologies)/DMEM or by incubation in 0.8 % trypsin (27250-018, Life Technologies)/DMEM for 15-30 min at 37°C. For dispase and trypsin treatment the subcutaneous fat was gently scraped off with a scalpel prior to incubation. Isolated epidermis was either directly snap frozen and stored at -80°C, or homogenized and further processed for RNA isolation (see below).

### RNA isolation and qRT-PCR

Total RNA from the epidermis of mice or from total skin was isolated with Trizol followed by purification with the RNeasy Mini Kit, including on-column DNase treatment (Qiagen, Hilden, Germany). Total RNA from cultured cells was extracted directly with the RNeasy Mini Kit. cDNA was synthesized using the iScript kit (Bio-Rad Laboratories, Berkeley, CA). Relative gene expression was determined using the Roche LightCycler 480 SYBR Green system (Roche, Rotkreuz, Switzerland).

The following mouse genes were probed with qRT-PCR primers: Ifit1, Irf7, Oasl2, Rps29, Stat1, Rsad2 and Stat2. The following human genes were probed with qRT-PCR primers: IFIT1, IRF7, RPL27, RSAD2, STAT1, STAT2 and OASL2.

### Immunofluorescence analysis of skin sections and cultured cells

Frozen mouse back skin sections were fixed with cold methanol, and unspecific binding sites were blocked with PBS containing 2% bovine serum albumin (BSA) (Sigma)/1% fish skin gelatin (Sigma)/0.05% Triton X-100 (Carl Roth GmbH, Karlsruhe, Germany) for 2 h at room temperature. Samples were then incubated overnight at 4°C with the primary antibodies (anti-IRF7 and anti-K14) diluted in the same buffer. After three washes with PBS-T (1×PBS/0.1% Tween 20 (Carl Roth GmbH)), slides were incubated at room temperature (RT) for 4 h with secondary antibodies (AF555-conjugated anti-rabbit IgG and AF488-conjugated anti-mouse IgG) and DAPI (4',6-diamidino-2-phenylindole dihydrochloride) (Sigma) as counter-stain, washed with PBS-T again and mounted with Mowiol (Hoechst, Frankfurt, Germany). Stained sections were photographed using a Leica SP1-2 confocal microscope equipped with a 63×0.6-1.32 NA (Iris) PL Apo Oil objective. Data acquisition was performed using Leica Confocal Software (Leica, Wetzlar, Germany). For immunofluorescence analysis of cultured cells, cells were washed in PBS and then either fixed for 5 min with cold methanol for ZO.1/IRF7 staining, or fixed in 4% paraformaldehyde (PFA) (Sigma) for 20 min at RT for Glyc-D staining. PFA fixed cells were then incubated for 10 min with 0.5% Triton X-100 in PBS. After 1 h blocking in PBS containing 2% BSA, cells were stained with primary antibodies for 1 h in the same blocking buffer. After three washes with PBS, cells were incubated with secondary antibodies (AF555-conjugated anti-mouse IgG, AF488-conjugated anti-mouse IgG and AF555-conjugated anti-rabbit IgG) and DAPI. Stained cells were photographed using a Zeiss Imager.A1 microscope equipped with an Axiocam MRm camera and EC Plan-Neofluar objectives (10x/0.3, 20x/0.5). For data acquisition we used the Axiovision 4.6 software (all from Carl Zeiss Inc., Jena, Germany).

### Preparation of protein lysates and Western blotting

Cells were harvested in T-PER tissue protein extraction reagent (Pierce, Rockford, IL) containing Complete Protease Inhibitor Cocktail (Roche). Lysates were cleared by centrifugation (13,000 rpm, 30 min, 4°C), snap frozen, and stored at -80°C. The protein concentration was determined using the BCA Protein assay (Pierce). Proteins were separated by SDS-PAGE and transferred onto nitrocellulose membranes. Membranes were incubated with primary antibodies and, subsequently, antibody-bound proteins were detected with horseradish peroxidase coupled antibodies against goat-IgG (Sigma), rabbit-IgG, or mouse IgG (both from Promega, Madison, WI).

### HSV-1 production and cell infection

HSV-1 was produced as described (Strittmatter et al., 2016). Sub-confluent HaCaT cells were starved overnight in serum-free medium and then incubated with HSV-1 (MOI = 0.5). Where indicated, infection was preceded by treatment with inhibitors (NSC23766 (50 µM), AZD4547 (1 µM) diluted in DMSO) or DMSO only for 8 h before treatment with FGF7 (15 ng/ml) or IFNα (1000 U/ml). Infected cells were left for 24 h before the assessment of viral replication (see below).

### Preparation of genomic human DNA and viral DNA from HSV-1 infected cells

Genomic and viral DNA was obtained from infected HaCaT cells using the HotSHOT genomic DNA preparation method (Truett et al., 2000) modified according to Strittmatter et al. (2016). Briefly, the supernatants of infected cells were removed, and 200 µl of alkaline lysis buffer (NaOH 25 mM, EDTA 0.2 mM) per plate of a 6-well plate were added to the remaining cells. The cells were scratched off and incubated in 1.5 ml Eppendorf tubes for 30 min at 95 °C. Then, the tubes were cooled down to 4 °C, and 200 µl of neutralization buffer (Tris- HCl 40 mM) were added before the samples were centrifuged (13000 rpm, 10 min, 4 °C) and the DNA concentration in the supernatant determined. Samples were used for quantitative PCR to measure HSV-1 replication. Primers for amplification of the genomic DNA for β-actin (5'-TAC TCC TGC TTG CTG ATC CAC-3' and 5'-TGT GTG GGG AGC TGT CAC AT-3') and viral glycoprotein B (GLYC-B) (5'-CGC ATC AAG ACC ACC TCC TC-3' and 5'-GCT CGC ACC ACG CGA-3') were used.

### Ex vivo HSV-1 infection

HSV-1 infection of mouse tail epidermis was performed as previously described (Rahn et al., 2015). Briefly, skin was prepared from the tails of 3 month-old mice, and afterwards the epidermis was separated from the dermis by dispase treatment (5 mg/ml). After floating the epidermal sheets on serum-free DMEM overnight, the epidermal sheets were incubated with HSV-1 alone (MOI = 2) or in combination with FGF7 (15 ng/ml), IFNα (1000 U/ml) and/or the inhibitors NSC23766 (50 µM) or AZD4547 (1 µM) for 48 h and subsequently fixed in 4% PFA for 1 h at RT. Then, tissues were incubated in blocking solution (PBS 2% BSA/1% fish skin gelatin/0.05% Triton Tx-100) for 2 h at RT and stained overnight at 4°C with an antibody against Glyc-D diluted in blocking solution. After 3 washes in PBS, epidermal sheets were incubated 4 h with AF555-conjugated anti-mouse IgG and DAPI diluted in PBS 0.05% Triton X-100 at RT and successively mounted with their basal side on top of a specimen slide, embedded in Mowiol and covered with coverslips. Stained samples were photographed and acquired as described for immunofluorescence analysis of cultured cells.

### LCMV infection and flow cytometry analysis

Sub-confluent HaCaT cells were starved overnight in serum-free medium and then incubated overnight at 37°C with the specified amounts of LCMV. Afterwards, HaCaT cells were detached from the 12-well plate using 1% trypsin and fixed/permeabilized in 500 µl 2x FACS Lyse (Becton Dickinson, Frankling Lakes, NJ) with 0.05% Tween 20 for 10 min at room temperature. The cells were washed, and intracellular staining was conducted for 30 min at room temperature using the LCMV nucleoprotein-specific antibody VL-4. After an additional washing step they were resuspended in PBS containing 1% PFA. Flow cytometry analysis was performed using an LSRII flow cytometer (Becton Dickinson). Raw data were analyzed using FlowJo software (Tree Star Inc, Ashland, OR).

### Cell transfection

The expression vectors pcDNA3.1-Rac1Q61L-HA and pcDNA3.1-EGFP-HA were kindly provided by Prof. Giorgio Scita (Firc Institute of Molecular Oncology, Milan, Italy). Immortalized mouse keratinocytes were seeded on 6-well plates (600'000/well), incubated for 24 h, and then transiently transfected with the aforementioned vectors using Lipofectamine 2000 reagent (Invitrogen) according to the manufacturer's protocols. After 24 h, cells were lysed in Trizol and TPER buffer for subsequent qRT-PCR and Western blot analysis.

### Luciferase Assay

Transient transfections of immortalized mouse keratinocytes were performed with the expression vectors for TK-Renilla and the expression vector pGL4.45[*luc2P*/ISRE/Hygro] (Promega), the latter containing five copies of an interferon-stimulated response element (ISRE) that drives transcription of the luciferase reporter gene. Cells were seeded into 12-well plates, cultured for 24 h, and transfected using Lipofectamine 2000. Subsequently, cells were starved overnight in serumfree medium, treated with FGF7 (10 ng/ml) and/or with IFNα (1000 U/ml) for 12 h, then lysed and assayed with a dual-luciferase assay system (Promega) according to the manufacturer's instructions. Relative light units were measured in a GloMax 96 microplate luminometer with dual injectors (Promega).

### Gene expression profiling and bioinformatics analysis

For gene expression profiling, epidermis from 9 K5-R1/R2 and 9 control mice at postnatal day 18 was separated from the dermis as previously described (Yang et al., 2010) and immediately frozen in liquid nitrogen. Samples from three mice per genotype were pooled and subjected to Affymetrix microarray hybridization (N = 3 per genotype) as previous described (Beyer et al., 2008). Genes, which were significantly and more than 2-fold up- or down-regulated in K5-R1/R2 compared to control mice were subjected to Ingenuity Pathway Analysis (Qiagen).

### Statistical analysis

Statistical analysis was performed using the PRISM software (Graph Pad Software Inc., San Diego, CA). Mann-Whitney U test for non-Gaussian distribution was used for experiments examining differences between two groups. **P*≤0.05, ***P*≤0.005, ****P*≤0.001.

### Example 2: Loss of FGF signaling induces expression of interferon-stimulated genes in keratinocytes in vivo and in vitro

To identify new FGF targets in the epidermis, mRNA expression profiling of epidermal sheets from mice lacking FGF receptors 1 and 2 in keratinocytes (K5-R1/R2 mice) and control animals (K5-cre) (Yang et al., 2010) was performed. The analysis was performed at P18 and therefore prior to the development of histopathological abnormalities to reduce the number of genes that are differentially regulated as a consequence of the phenotype. Bioinformatics analysis revealed that 124 genes were down- and 175 genes were upregulated in K5-R1/R2 mice compared with controls (f.c. > 2, **p* ≤ 0.05). Using Ingenuity Pathway Analysis to identify FGF-regulated functional pathways, it was found that most of the modulated genes are involved in the type I interferon (IFN) response. In particular, expression of various Interferon-Stimulated Genes (ISGs), such as interferon regulatory factor 7 (*Irf*7), radical S-adenosyl methionine domain containing 2 (*Rsad2*), signal transducer and activator of transcription 1 (*Stat1*) and 2 (*Stat2*), 2'-5' oligoadenylate synthetase-like 2 (*Oasl2*) and interferon-induced protein with tetratricopeptide repeats 1 (*Ifit1*) was strongly up-regulated in the epidermis of FGFR1/R2-deficient mice. These data were confirmed by quantitative PCR (qRT-PCR) analysis of RNA from epidermal samples of K5-R1/-R2 and control mice at the age of 3 months (Fig. 1A). Since loss of FGFR1 and FGFR2 in keratinocytes caused an early accumulation of mast cells in the dermis and of γδ T cells in the epidermis, which started at the age of P9 (Sulcova et al., 2015a; Sulcova et al., 2015b), qRT-PCR was performed using RNAs from epidermis of young mice (postnatal day 9 (P9) and P5) to exclude a potential role of the progressive increase in immune cells in the regulation of ISGs. However, the up-regulation of ISGs, such as *Rsad2* and *Oasl2*, was already significant at P5 and very robust at P9 (Fig. 1B, C), demonstrating that the ISG overexpression in K5-R1/R2 mice is an early effect and precedes the accumulation of immune cells in the skin. Surprisingly, this was not associated with a significant up-regulation in the mRNA levels of the different types of IFNα, of IFNβ, or of IFNγ in K5-R1/R2 in the epidermis or total skin (data not shown), suggesting that FGFR signaling modulates the tonic IFN signaling that occurs in many cell types (Gough et al., 2012). Up-regulation of ISGs was also seen at the protein level by immunofluorescence analyses, which revealed a pronounced increase in IRF7 in the nuclei of basal and suprabasal keratinocytes of 3 month-old K5-R1/R2 mice compared with control animals (Fig. 1D). Hence, it is demonstrated that *in vivo* loss of FGFR signaling increases the expression of ISGs in keratinocytes, both at the RNA and the protein level. This is a cell autonomous effect, since expression levels of *Irf7, Stat2, Oasl2* and *Ifit1* were also significantly higher in cultured primary keratinocytes from K5-R1/R2 mice compared to cells from control mice (Fig. 2A).

### Example 3: FGF7 suppresses transcription of interferon-stimulated genes in keratinocytes

It was tested if FGF7, a major ligand for the b splice variants of FGFR1 and FGFR2 expressed by keratinocytes (Zhang et al., 2006; Werner et al., 1993), directly suppresses ISG expression in cultured keratinocytes. Indeed, when keratinocytes from wild-type mice were treated with recombinant FGF7, expression of different ISGs was rapidly suppressed (Fig. 2B). Western blot and immunofluorescence analysis confirmed the FGF-mediated down-regulation of IRF7 at the protein level (Fig. 2C and D). To investigate if this occurs at the transcriptional level, spontaneously immortalized mouse keratinocytes (Meyer et al., 2012) were transfected with the expression vector pGL4.45[*luc2P*/ISRE/Hygro] that contains a luciferase reporter gene under control of an interferonstimulated response element (ISRE), which controls the expression of most ISGs (Gough et al., 2012). Thus, activation of the type I interferon receptor induces tyrosine phosphorylation of STAT1 and STAT2, which, together with IRF9, form the transcription complex ISGF3 that binds and activates the ISRE in the promoters/enhancers of ISGs (Darnell et al., 1994). As expected, IFNα stimulation of IRES-Luc-transfected immortalized mouse keratinocytes resulted in a significant increase in luciferase activity (Fig. 3A). In contrast, FGF7 treatment reduced the luciferase activity compared with untreated samples (Fig. 3A). Remarkably, the stimulation of luciferase expression by IFNα was completely suppressed in the presence of FGF7 (Fig. 3A), indicating that FGF7 is a strong inhibitor of the ISRE-mediated transcriptional activity - even in the presence of its canonical activator IFNα. Consistent with this finding, the increase in ISG expression that occurred in mouse keratinocytes in response to IFNα treatment was suppressed in the presence of FGF7 (Fig. 3B). The effect of FGF7 on ISG expression was then studied in the presence of polyinosinic-polycytidylic acid (poly(I:C)), a potent synthetic Toll-Like Receptor 3 (TLR3) activator (Matsumoto & Seya, 2008). TLR3, a double-stranded RNA sensor, has an important role during viral infections, since TLR3 signaling leads to over-expression of interferons and thus to the induction of ISGs (Matsumoto & Seya, 2008). Consistent with previously published data (Tohyama et al., 2005; Dai et al., 2006), poly(I:C) induced ISG expression in keratinocytes (Fig. 3C). However, this was suppressed upon co-treatment with FGF7 (Fig. 3C). The inhibition of ISRE-mediated gene expression by FGF7 was confirmed for human HaCaT keratinocytes, demonstrating the general importance of this pathway across species (Fig. 7). Hence, our data identify FGF7 as a new and extremely potent inhibitor of IFN-mediated expression of antiviral genes.

### Example 4: FGFs control ISG gene expression in keratinocytes via Rac1 and p38

To identify the molecular mechanisms involved in the FGF-mediated suppression of ISG expression, the consequences of pharmacological inhibition of various intracellular components of the FGF signaling cascade was examined. Among different tested compounds that are known to inhibit FGFR-mediated signaling pathways, only a RAC1 inhibitor (NSC23766) and a P38 inhibitor (SB203580) rescued the FGF7-mediated ISG down-regulation (Fig. 4A and B), while inhibitors of PI3 kinase, Akt1/2, and Erk1/2 had no effect (data not shown). An efficient rescue was also observed with AZD4547, a novel and selective inhibitor of the FGFR1, FGFR2 and FGFR3 kinase activities (Gavine et al., 2012) (Fig. 4C), demonstrating that the effect of FGF7 on ISG expression is mediated via FGFR kinase activity. To further confirm the role of RAC1 in ISG modulation, we expressed a constitutively active mutant of RAC1 (Q61L mutation) in immortalized mouse keratinocytes (Fig. 4D, left panel). RAC1-Q61L indeed mimicked the effect of FGF7 stimulation and caused a significant down-regulation of the analyzed ISGs (Fig. 4D, right).

### Example 5: FGF signaling promotes herpes simplex virus 1 replication in keratinocytes in vitro and ex vivo

The potent effect of FGF signaling on ISG expression raised the intriguing question if modulation of FGF signaling can affect viral replication. To test this possibility, viral infection studies were performed using exponentially growing HaCaT cells, which represent *bona fide* undifferentiated human keratinocytes (Boukamp et al., 1988). Since keratinocytes are the first entry sites for herpes simplex virus type 1 (HSV-1) (Petermann et al., 2015), it was decided to use this pathogen to investigate the possible role of FGF signaling in viral infection. Sub-confluent HaCaT cells were infected with HSV-1 (MOI = 0.5) and concomitantly left untreated or treated with FGF7. As control, infected cells were incubated with IFNα. After 24 h, the amount of viral glycoprotein B (Glyc-B) DNA was determined in the infected cells by qPCR to assess the extent of HSV-1 replication (Strittmatter et al., 2016). To allow comparisons between samples, Glyc-B DNA levels were normalized to host P-actin DNA levels. We observed a robust increase in viral replication by FGF7, while, as expected, IFNα significantly reduced the amount of HSV-1 DNA (Fig. 5A). This was confirmed by immunofluorescence analysis of the infected cells for glycoprotein D (Glyc-D), another important HSV-1 protein. As shown in Fig. 5B, the number of Glyc-D-positive fused cells was significantly higher in FGF7 treated samples, and the size of these cells was also increased. The strong effect of FGF7 on viral replication was even markedly visible in live cells without immunofluorescence staining. Thus, within 48 h after viral infection, HSV-1 infected cells had fused, but were still attached to the dish. In the presence of FGF7, however, infected cells were completely detached and floating, indicating acceleration of the viral life cycle in the presence of FGF7 (Fig. 5C). As expected, IFNα treated HSV-1 infected cells were almost indistinguishable from non-infected cells (Fig 5D). These results identify FGF7 as a potent accelerator of HSV-1 infection in keratinocytes. Importantly, this effect is not restricted to HSV-1 as revealed by infection studies with Lymphocytic Choriomeningitis Virus (LCMV), a murine pathogen, which can also infect human cells (Welsh & Seedhom, 2008). When HaCaT cells were infected with 0.04, 0.2 and 0.5 MOI of LCMV and immediately exposed to FGF7, expression levels of LCMV nucleoprotein (NP) were strongly increased in the presence of the growth factor as assessed by flow cytometry (Fig. 5D). To unravel the importance of the FGFR/Rac1 signaling pathway for the viral life cycle, HSV-1 infected HaCaT cells were treated with FGF7 in the presence or absence of the Rac1 inhibitor NSC23766 or the FGFR kinase inhibitor AZD4547. After 24 h, Glyc-B DNA levels were drastically reduced in NSC23766 treated cells, even in the presence of FGF7, indicating a role of Rac1 in viral replication (Fig. 6A). Surprisingly, the effect of NSC23766 was stronger than the effect of IFNα, which was used as a positive control (Fig. 6A). A very strong reduction of Glyc-B expression was also observed in AZD4547 treated samples (Fig. 6A). As expected, the FGFR kinase inhibitor inhibited the positive effect of FGF7 on viral replication. Remarkable, however, it even suppressed HSV-1 replication in the absence of exogenous FGF7 (Fig. 6A), suggesting that the low levels of autocrine FGF signaling that occur in keratinocytes (data not shown) promote HSV-1 replication. These results were verified by immunofluorescence staining for Glyc-D. Thus, NSC23766 treatment strongly reduced the amount and size of Glyc-D-positive fused cells similar to IFNα treatment, while the effect of and AZD4547 was weaker, but still statistically highly significant (Fig. 6B). Finally, the effect of FGF7 on HSV-1 infection/replication in a more complex system was investigated, using a recently published protocol to infect epidermal sheets from mouse tail skin *ex vivo* (Rahn et al., 2015). Epidermal sheets were incubated with HSV-1 alone or in combination with FGF7 and the inhibitors NSC23766 or AZD4547. After 48 h, tissues were stained with the Glyc-D antibody to monitor the infection. In the absence of FGF7, HSV-1 preferentially infected cells of the hair follicles, while in the presence of FGF7, viral infection was strongly promoted and was also observed throughout the interfollicular epidermis (Fig 6C). Viral dissemination was potently suppressed by NSC23766 and by AZD4547 (Fig 6C), thus confirming the *in vitro* data. Overall, these results revealed that FGF signaling is able to promote the viral life cycle in keratinocytes *in vitro* and *ex vivo* through a Rac1-dependent signaling pathway. Most important, they show the utility of FGFR inhibitors for treatment of viral infections in the skin.

## Claims

1. Compound for inhibiting (i) FGFR kinase activity or (ii) a component of the FGFR kinase signaling pathway for use in the treatment of viral infections.

2. Compound for use according to claim 1, wherein the compound (i) for inhibiting FGFR kinase activity is selected from the group consisting of
(a) FGFR kinase inhibitors: AZD4547, Ponatinib, Dovitinib, Nintedanib, Lenvatinib, Lucitanib, Brivanib, ENMD-2076, BGJ398, FGF401, Lucitanib, PD173074, SU5402, SSR128129E, ARQ 087, LY2874455, Debio 1347, TAS-120, Erdafitinib, Nintedanib, and Orantinib; FGFR ligand traps: FP1039; and FGFR neutralizing antibodies: IMC-A1, PRO-001, R3Mab, FPA144 and MGFR1877S;
(b) preferably AZD4547, BGJ398, LY2874455, Debio 1347, TAS-120, Erdafitinib ,FPA144 and FP1039;
(c) more preferably AZD4547 and BGJ398; and
(d) a physiologically acceptable salt of (a), (b) and (c).

3. Compound for use according to claim 1, wherein the compound for (ii) inhibiting a component of the FGFR kinase signaling pathway is selected from the group consisting of
(a) RAC1-inhibiting compounds, preferably selected from the group consisting of NSC23766, EHop-016, Azathioprine, EHT 1864;
(b) p38 MAPK-inhibiting compounds, preferably selected from the group consisting of SB203580, VX-702, VX-745, Pamapimod, losmapimod, Dilmapimod, Doramapimod, BMS-582949, ARRY-797, PH797804, SCIO-469, SD-0006, AMG-548, LY2228820, SB239063, Skepinone L, SB202190 and TAK715; and
(c) a physiologically acceptable salt of (a) and (b).

4. Compound for use according to any of claims 1 to 3, wherein
(a) the compound is selected from the group consisting of AZD4547, BGJ398, FP1039, FPA144 and physiologically acceptable salts thereof; and
(b) the viral infection is caused by a virus selected from the group consisting of Herpes simplex virus 1 and/or 2, Human Papilloma viruses, Ebola virus, Marburg virus, Venezuelan Equine encephalitis virus, Chikungunya virus, Easter Equine Encephalitis virus, Western Equine Encephalitis virus, Monkey Pox virus, Corona virus, Respiratory Syncytial virus, Adenovirus, Human Rhinovirus, Influenza virus, HIV, HCV, Norovirus, Saporovirus, cytomegalovirus (CMV), Dengue virus, West Nile virus, Yellow fever virus, Zika Virus, and HBV.

5. Compound for use according to any of claims 1 to 3, wherein
the compound is selected from the group consisting of **FGFR kinase inhibitors:** AZD4547, Ponatinib, Dovitinib, Nintedanib, Lenvatinib, Lucitanib, Brivanib, ENMD-2076, BGJ398, FGF401, Lucitanib, PD173074, SU5402, SSR128129E, ARQ 087, LY2874455, Debio 1347, TAS-120, Erdafitinib, Nintedanib, and Orantinib; **FGFR ligand traps:** FP1039; and **FGFR neutralizing antibodies:** IMC-A1, PRO-001, R3Mab, FPA144, and MGFR1877S, and physiologically acceptable salts thereof;
and the viral infection is caused by a virus selected from the group consisting of Dengue virus, HSV1, HSV2, HIV1, HIV2, HCV, Zika Virus, Influenza virus, SARS virus or MARS virus.

6. Compound for use according to any of claims 1 to 3, wherein the compound is selected from the group consisting of AZD4547, BGJ398 and physiologically acceptable salts thereof; and the viral infection is caused by a virus selected from the group consisting of HSV1, HSV2 and Zika Virus.

7. Compound for use according to any of claims 1 to 3, wherein the compound for use is
- (i) a compound for inhibiting FGFR 1, FGFR 2, and/or FGFR 3 kinase activity, or a compound for inhibiting FGFR 1, FGFR 2, and/or FGFR 3 kinase signaling, for the treatment of a viral disease in epithelial cells of the skin (keratinocytes), preferably an HSV1 or HSV2 infection in keratinocytes;
- (ii) an FGFR ligand trap binding ligands of FGFR2b for the treatment of a viral disease affecting epithelial cells of the skin (keratinocytes), preferably an HSV1 or HSV2 infection of keratinocytes, or for the treatment of a viral disease affecting the lung, preferably an influenza virus infection of the lung;
- (iii) a compound for inhibiting FGFR 1, FGFR 2, FGFR 3 and/or FGFR 4 kinase activity, or a compound for inhibiting FGFR 1, FGFR 2, FGFR 3 and/or FGFR 4 kinase signaling for the treatment of a viral disease affecting T cells, preferably an HIV infection of T cells; or
- (iv) a compound for inhibiting FGFR 1, FGFR 2, FGFR 3, and/or FGFR 4 kinase activity, or a compound for inhibiting FGFR 1, FGFR 2, FGFR 3 and/or FGFR 4 kinase signaling for the treatment of a viral disease in hepatocytes, preferably an HCV or HBV infection in hepatocytes.

8. Compound for use according to any of claims 1 to 3, wherein the compound is a compound for inhibiting FGFR 1 and FGFR 2 kinase activity, or a compound for inhibiting FGFR 1 and FGFR 2 kinase signaling, preferably FP1039, FPA144, AZD4547 or BGJ398, for use in the treatment of a viral disease in keratinocytes, preferably an HSV1 or HSV2 infection in keratinocytes.

9. Pharmaceutical composition comprising at least one compound for use according to any of claims 1 to 8 and optionally further physiologically acceptable excipients.

10. Pharmaceutical composition for use according to claim 9, wherein the pharmaceutical composition is for topical, oral, intravenous, intranasal, or rectal administration.

11. Pharmaceutical composition for use according to claim 9 or 10, wherein the at least one compound is selected from the group consisting of AZD4547, BGJ398 and physiologically acceptable salts thereof; the composition is for oral, intranasal, intravenous, intramuscular, intradermal, subcutaneous, intraperitoneal or topical administration, preferably topical administration; and the composition is for use in the treatment of HSV-1 inventions.

12. Compound or composition for use according to any of claims 1 to 11, wherein the viral infection is a HSV 1 infection of keratinocytes and the compound or composition is preferably for topical administration.

13. Compound or composition for use according to any of claims 1 to 11, wherein the compound or composition is for use in the treatment of a viral infection in a human or animal, preferably a human, mammal or bird, more preferably a human.

14. Method for the therapeutic or prophylactic treatment of a viral disease, preferably a viral disease caused by a virus selected from the group consisting of Herpes simplex virus 1 and/or 2, Human Papilloma viruses, Ebola virus, Marburg virus, Venezuelan Equine encephalitis virus, Chikungunya virus, Easter Equine Encephalitis virus, Western Equine Encephalitis virus, Monkey Pox virus, Corona virus, Respiratory Syncytial virus, Adenovirus, Human Rhinovirus, Influenza virus, HIV, HCV, Norovirus, Saporovirus, cytomegalovirus (CMV), Dengue virus, West Nile virus, Yellow fever virus, Zika Virus, and HBV, comprising the steps of:
(a) providing a compound according to any of claims 1 to 8 or a composition according to any of claims 9 to 13; and
(b) administering the compound or composition of (a) to the subject in need thereof in a pharmaceutically effective amount, preferably by oral, intranasal, intravenous, intramuscular, intradermal, subcutaneous, intraperitoneal or topical administration, more preferably by topical administration.

15. The method according to claim 14, wherein the subject is selected from the group consisting of a human and an animal, preferably a human, mammal and bird, more preferably a human.
